# EUROPEAN PATENT APPLICATION

(11) **EP 1 684 556 A2**
(43) Date of publication of application: **26.07.2006**
(21) Application number: 06111770.1
(22) Date of filing: 21.01.1997
(51) Int. Cl.: H05F 7/00

(54) **Environment improving implement and environment improving method**

(30) Priority: 26.06.1996 JP 16632796; 09.05.1996 JP 11510496
(62) Divisional of application: 97100825.5
(71) Applicant: Takamatsu, Kuniaki, Kaminoyama-shi, Yamagata 999-3243 (JP); Ohara, Toyoko, Nara-shi, Nara 631- 0033 (JP)
(72) Inventor: Takamatsu, Kuniaki, Yamagata, 999-3243 (JP); Ohara, Toyoko, Nara-shi Nara, 631-0033 (JP); Kikuchi, Hideaki, Osaka-shi, Osaka 535 (JP)
(74) Representative: Dallmeyer, Georg

(57) **Abstract**

An environment improving implement wherein a predetermined granular and/or powdery element and/or compound is sealed in a glass tube having a predetermined shape. The element and/or compound is selected from among Si, SiOₓ, C, Al, P, Ge, Sn, Pb, Cu, Ag, Au, Ni and Fe. When the environment improving implement is embedded underground, a stray current is ready to flow, thereby enabling to prevent electrification. When a sounding body is sounded in a space defined by at least three environment improving implements, air, humaix bodies, animals, plants and objects can be activated. The environment improving implement cleans water, air and soil only by disposing.

## Description

The invention relates to an environment improving implement and an environment improving method, wherein minus ions are used for improving environment by removing static electricity.

It is generally known that stray current flows in the atmosphere and the earth. The intensity varies depending on changes in atmospheric potential caused by changes in weather, geographic features, and other natural conditions, and also depending on artificial things such as electric apparatuses, railways, power cables, and so forth and is influenced with metalliferous veins, ground water arteries, and underground cables, if they are present. Ordinarily, in the ground with a height above the sea of 100 m, earth current with all intensity of about 15 mu A flows. However, in the modern society, with the spread of electric apparatus, the intensity of the stray current has been higher almost in any place. This appears as static electricity which is electrified on the earth. In event that earth current having an intensity of 30 to 40 mu Λ flows through a human body to electrify, there is a great possibility that the current causes various hindrances to men's health. Light symptoms caused by the electrification include stiffness in the shoulder, headache, fatigue feeling, and so forth. However, if the electrification lasts in a long term, or the charge quantity is high, the morbid symptoms become severe. In some cases, their human relations are badly influenced with the poor physical conditions.

The electrification exerts a bad influence not only over human bodies but also over plants, animals, and even objects. The bad influences can be appreciated as follows; for plants, the growing rate is decreased, the vitality of forests is deteriorated, and for animals, the vitality is reduced, and the morbid symptoms appear. In an electrified condition, cat fish can hardly foreknown an earthquake, cat fish in the river being well-known as an animal having ability to foreknow an earthquake in Japan.

Though the electrostatic charge, generated by the electrification of an object, is effectively used in some cases, they may cause the following hindrances: mechanical hindrances occur, that is, electrified objects such as powder, fibers, sheets, and so forth get to be caught in other objects, combustible and explosive objects are ignited when static electricity is discharged, which may induce fires and explosions; and the electrostatic charge may be a cause of the breaking, error action, quality-deterioration of precision apparatuses such as semiconductor parts, and so forth.

Conventionally, as a method of inhibiting the residence of earth current to prevent the electrification in a wide area, the embedment of carbon is carried out. Hereinafter, the embedment of carbon will be described.

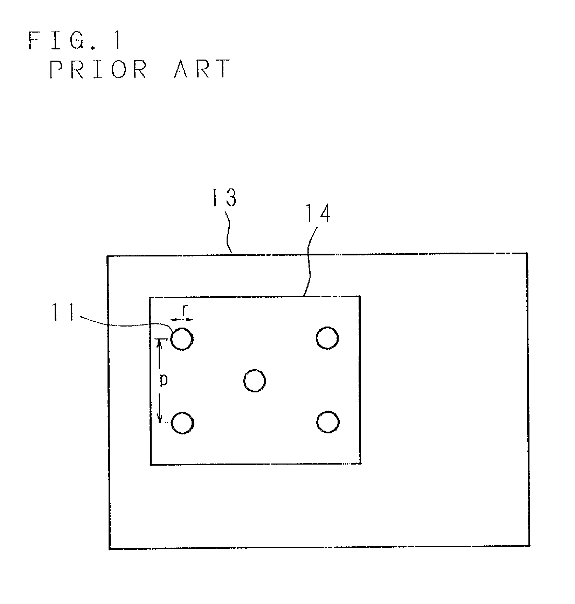

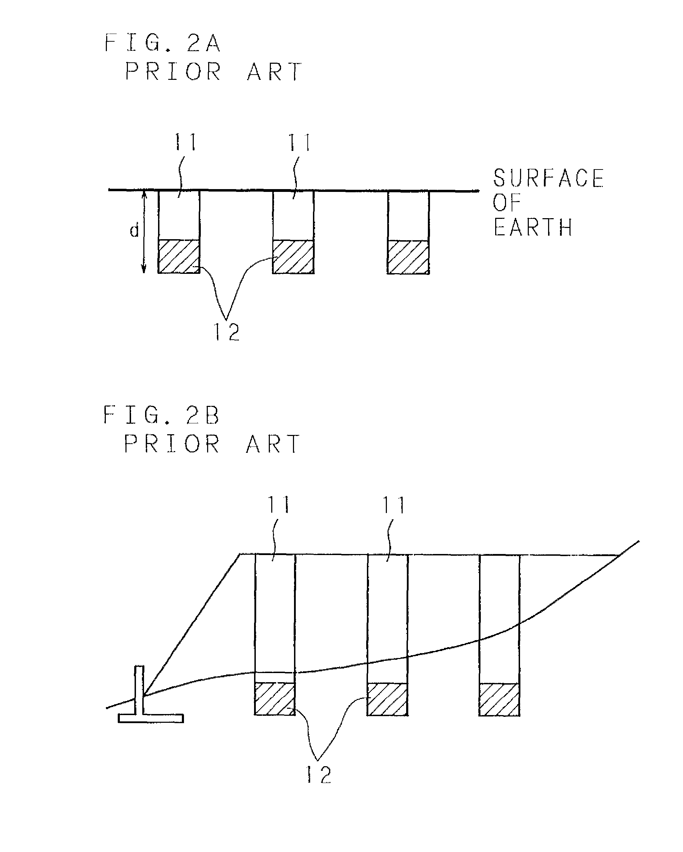

For example, as shown in FIG. 1, for a building lot with a land area of 100 tsubo (1 tsubo 3.3m<2>) and a building area of 40 tsubo, embedment pits 11 with a diameter (r) of 1 m and a depth (d) of 1 to 2 m are dug at intervals (p) of 5 to 10 m. 200 to 500 kg per pit of carbon 12 (for example, coconut shell carbon) is embedded into a pit. The carbon 12 may have a form of grains or powder, depending on the conditions of the land 13 and the building 14. The intervals between the embedment pits 11 can be changed depending on the conditions of the land and the building FIGS. 2A and 2B are sectional model views of the embedment pits 11. FIG. 2A shows pits provided in a level ground. The pits shown in FIG. 2B are provided in a sloping land (the building lot is an embankment). In the case of such sloping lands as shown in FIG. 2B, the carbon 12 is embedded in the mountain-shape land at the same horizontal-level.

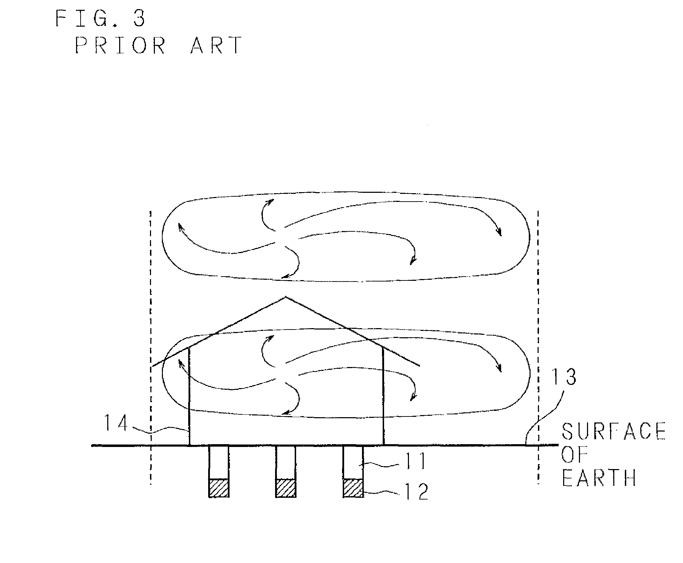

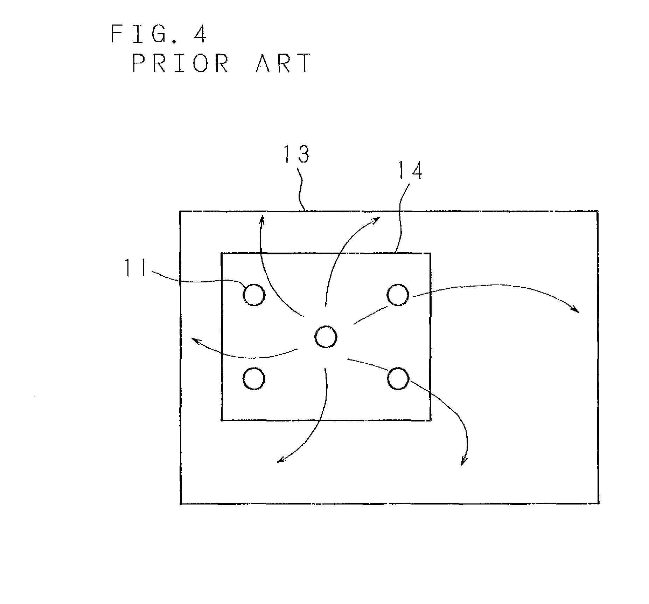

When the carbon is embedded, the electric resistance is reduced as a whole, so that the stray current flows, swirling and spreading as shown in FIG. 3 (side view) and FIG. 4 (plan view). This reduces the electrification of men, plants and animals, and objects present in the land in which the carbon is embedded, that is, reduces the static electricity on the ground. Such phenomena are effective to a height above the sea level of about 3 m, which serves for the stabilization of them.

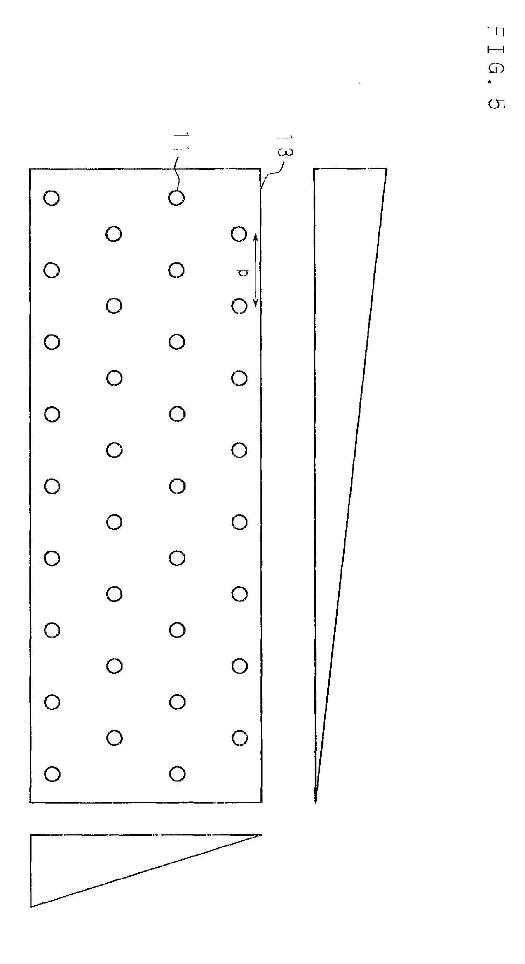

FIG- 5 is a plan view which illustrates the application to a factory and a farm. The embedment pits 11 for embedding the carbon 12 have an interval (p) of 10m, are provided in the sites positioning at the apexes of a triangle. The embedment quantities of the carbon 12 are controlled according to the potential inclination. In the case of the potential inclination to be provided in the lateral direction as shown in FIG. 5, 200 kg of the carbon 12 is embedded in the embedment pit 11 in the highest potential site, and 500 kg of the carbon 12 is done in the embedment pit 11 in the lowest potential site. Between both sites, the quantities of the carbon to be embedded are determined according to the proportional distribution. Also, in the case of the potential inclination in the longitudinal direction of the drawing, the quantities of the carbon to be embedded are controlled. The embedment pits 11 have a diameter of 1 to 1.2 mm and a depth of 1 to 2 m. In this case, it is desirable that the carbon 12 is embedded in the mountain-shape land at the same horizontal level. The case where the carbon embedment is applied to the factory and farm can also obtain a similar effect to the case of residential land.

When the embedment of the carbon is carried out, 1 to 2 tons of the carbon 12 is required for a house. For the application to a factory (lot 1000 tsubo, building 500 tsubo), 10 to 30 tons of the carbon 12 is needed. In addition, many embedment pits 11 are dug. The overall amount of money including the construction cost is vast. The economical burden is very heavy.

When carbon is sprayed on the ground surface to a thickness of about 3 to 10 cm, effects to some degree can be obtained. However, the effects are lower as compared with the underground embedment, and the effectiveness-lasting time is short, that is, about half a year.

The influence of static electricity is more apparently recognized indoor, as compared with the outdoor condition. For the purpose of making indoor air more comfortable or activating the indoor air, air cleaning devices having function for generating minus ions, infrared ray generators, static-electricity removing devices, dehumidifiers, humidifiers, and so forth have been rapidly spread. In addition, bedding (futon, pillows), clothes, cosmetics, and so forth which allow minus ions to act on human bodies have been practically used.

The effects of minus ions can be explained based on the biological action of atmospheric ions or the principle of the ion introduction.

The causes of the atmospheric ions include ionization effects by space rays, ultraviolet rays, natural radioactive substances, atmospheric discharge (lightning), and so forth, and triboelectricity generated at snow falling, effects accompanied by the changes in surface area of water drops, the Lennard effect, and so forth. Also, the phenomenon is called global circuit wherein minus ions flow between ionosphere and earth circularly, and current flows in the opposite direction. It is generally thought that as the biological actions of the atmospheric ions generated by the above-mentioned causes, the minus ion alkaline atmosphere has systematic actions such as tranquil, invigorating, hypnotic, appetite promoting actions, and so forth, blood pressure drop action, blood sugar reduction, vasodilatation, diuretic action, and so forth.On the other hand, it is generally thought that the plus ion acidic atmosphere has systematic actions such as excitable, unpleasant feeling, sleeplessness, appetite diminishment, and so forth, blood pressure rising action, blood sugar increasing action, vasoconstriction, diuretic inhibition action, and so forth.

The principle of the ion introduction is that various substances are ionized by the application of electrolytic action, and through the ions an objective substance itself is permeated into subcutaneous tissues. It is generally thought that when the activated oxygen is absorbed into a layer near the skin surface, the acidic horny layer is neutralized to be softened and the metabolism of the skin becomes active, recovering the skin function to its normal state.

In addition, the minus ions enhance the blood flow in the skin, adjusts the function of the autonomic nervous system, makes the blood alkaline so as to activate the functions of the cells. Thus, the minus ions has a positive action to prevent the skin aging and dissolve other troubles. Moreover, the minus ions, normalizing the skin functions, are very effective in prevention of the loss of hair and hair restoration.

In the indoor condition, minus ions, when they are generated with a device having a minus ion generating function, neutralizes the plus charge and further can convert to the minus ion environment. However, when the removal of electrostatic charge or the activation of a space is attempted with an electric apparatus, there exists such a problem that the electrostatic charge quantity is increased, due to the fact that the electric apparatus itsel is charged, even though the air is cleaned.

Moreover, it is desired to clean water and air which are indispensable to the preservation of our life.

Conventionally, drink water is obtained by treating river water and groundwater (raw water) to clean, if necessary. As means for treating water to clean, (1) the flocculation of suspended matters, and the solid - liquid separation, (2) the coagulation of dissolved matters, oxidation, biochemical modification, adsorption, and ion exchange, (3) the agglutination and filtration of bacteria and virus, and their disinfection, (4) the neutralization of acids and alkali, and so forth are exemplified, and are employed in combination of them depending on the uses.

In general, underground water, not containing objects to be removed, has good water qualities. Therefore, the underground water, after it is subjected to chlorine disinfection only, is supplied. On the other hand, for river water and lake water, viscosity colloids, algae plankton, and contaminants deriving from the nature are objects of the water cleaning. For this purpose, the slow sand filtration or quick sand filtration system is employed.

According to the slow sand filtration system, in addition to the mechanical filtration of suspended matters, the oxidation, adsorption and decomposition of ammonia, iron, manganese, organic components, and smell components with microorganisms propagating in the surface layer, the predation with water bacteria, and the stabilization of nitrogen components with bacteria in the sand layer can be achieved.

According to the quick sand filtration system, viscosity colloids and color colloids in water are flocculated by the addition of a coagulant having a positive charge, and then the flocculation is precipitated and filtrated to be separated. As the coagulant, aluminium salts are mainly used. In part, iron salts and high polymeric substances are used. Even by the quick sand filtration system, dissolved contaminants can be flocculated, precipitated, and filtrated to be cleaned to some degree, using an oxidizing agent, the absorption into aluminium hydroxide, and the coagulation. For extreme contamination and bad smells, the oxidization with ozone and the adsorption to active carbon are further used in some cases.

Water with physiological safety for drinking or tasteful water contains minerals dissolved out of rocks and stones in an appropriate amount. In the case of ultra-pure water required, processes such as ultra fine filtration, multi-step reduced-pressure distillation, ion exchange, reverse osmosis, and so forth are used to produce pure water on a required water-quality level. As described above, many water cleaning methods suitable for the respective uses of water have been developed.

In recent years, with the more contamination of raw water, the amounts of chlorine and chlorinated lime used in the above-described processes have been increased. However, there have been more people who are sensitive to the taste and smell of the chlorine and the chlorinated lime and dislike them. For this reason, water cleaning devices for cleaning tap water in their homes have been widely spread. There are various types of such water cleaning devices. That is, ones are attached to taps, and others are placed in the cabinets of sinks, and so forth. However, the water cleaning devices to be attached to taps have such problems that the taps become heavy to be handled, persons using tap water can not sufficiently see the working space under the water cleaning devices. For the water cleaning devices to be placed in the cabinets of sinks, there are such problems that the spaces for working in the cabinets are reduced. For both types of the water cleaning devices, it is necessary to maintain them, e.g., exchange the fillers.

For preventing of indoor bad smells, deodorants using active carbon and so forth, deodorizing sprays, perfumes for emitting various scents, and so forth are available. With conventionally used deodorants and deodorizing sprays, it is difficult to deodorize smells sufficiently. As to perfumes, perfumes are liked or disliked by different persons. In some cases, persons feel that perfumes smell bad.

In addition, air conditioners having an air cleaning function have been spread. However, their spread is inhibited because the devices are too expensive, the large-scale attachment is necessary, and for use of the devices, electricity is required.

The present invention has been devised to solve abovementioned problems. A main object of the invention is to provide an environment improving implement and an environment improving method with use of minus ions.

The first form of this invention is to provide an electrification preventing device and a method of preventing electrification which are applicable to reduce electrification of static electricity due to stray current easily, using relative inexpensive means, and prevent a human body, a plant, an animal, and an object from being electrified.

An electrification preventing device of the invention is characterized in that a predetermined granular and/or powdery element and/or compound is sealed in a glass tube. Said element and/or compound may be Si and/or SiOx (0<x</=2). Or said element may be selected from the group consisting of C, Al, P, Ge, Sn, Pb, Cu, Ag, Au, Ni, or Fe.

A method of preventing electrification of the invention is characterized in that the electrification preventing device is embedded underground in the vertical direction.

The second form of this invention is to provide a space activating device and a method of activating a space, capable of activating a space merely by placement of the device, not using electricity.

A space activating device of the invention is characterised in that it contains a glass container having a point portion, and granular and/or powdery Si and/or SiOx (0< x</=2), or element selected from the group consisting of C, Al, P, Ge, Sn, Pb, Cu, Ag, Au, Ni, or Fe is charged in the glass container.

The glass container can have a columnar shape in the lower side and a conical shape in the upper side.

A space activating method of the invention is characterized in that the space activating devices are arranged in at least three positions defining a space to be activated with the point portions oriented upward, a sounding body is sounded in the defined space, and is rotated clockwise several times and then rotated anti-clockwise several times.

The sounding body may be sounded while the space activating device is further arranged near the center of the defined space.

The sounding body may be a tuning fork.

Minus ions emitted from the silicon in the glass container are filled into the space defined by the at least three space activating devices to form a barrier. The minus ions act on the air, living things such as human bodies, plants, animals, and so forth, and objects to activate. With waves generated from the sounding body, the activation state is maintained.

The third form of this invention is to provide a cleaning/deodorizing bar of which the effect is long-lasting only by disposing, not substantially requiring a further space.

Minus ions activate the cells and liquids constituting living things, and hydrogen in water and air. Therefore, this invention utilizes the minus ions in order to clean water and air.

A cleaning/deodorizing bar of the invention is characterized in that a predetermined, granular and/or powdery element and/or compound, for example, Si, SiOx (0<x</=2), C, Al, P, Ge, Sn, Pb, Cu, Ag, Au, Ni, or Fe is sealed in a glass tube of which one end is substantially conical and the other end is plane.

It is presumed that minus ions are emitted from the element or compound sealed in the glass tube, and activates the hydrogen in the air. Minus ions emitted through the substantially conical top end of the glass tube act on smells, and minus ions emitted through the plane top of the glass tube act on water and earth.

When opposite ends of the glass tube have a substantially conical shape, minus ions emitted from the ends acts on smells and wind.

When opposite ends of the glass tube are substantially plane, minus ions emitted through the top ends act on water and earth.

Higher effects can be obtained as the size of the glass tube is larger in any cases. The size can be determined according to the purpose.

It is preferred to use an element or compound minus ionized previously. Higher effects can be obtained as the ionization degree is higher.

The above and further objects and features of the invention will more fully be apparent from the following detailed description with accompanying drawings.
FIG. 1 is a plan view showing a case of an application of carbon embedment to a residential land;
FIG. 2A is a schematic sectional view showing embedment pits provided in a level ground;
FIG. 2B is a schematic sectional view showing embedment pits provided in a sloping land;
FIG. 3 is a side view explaining air discharge of a stray current;
FIG. 4 is a plan view explaining air discharge of the stray current;
FIG. 5 is a plan view explaining an application of carbon embedment to a factory or a farm;
FIG. 6 is a schematic view showing an electrification preventing implement of the invention;
FIG. 7 is a side view of a house in which the electrification preventing implement shown in FIG 6 is embedded and its neighborhood;
FIG 8 is a plan view of the house and its neighborhood;
FIGS. 9A and 9B are perspective views showing space activating implements of the invention;
FIG. 10 is a plan view of glass containers mounted in a motorcar;
FIG. 11 is a plan view of glass containers disposed indoors;
FIG. 12 is a schematic view showing a cleaning/deodorizing bar of the invention;
FIG. 13 is a perspective view which shows that the cleaning/deodorizing bar shown in FIG. 12 is a attached to a valve at a tap of a water supply;
FIG. 14 is a perspective view showing a deodorizing bar of the invention;

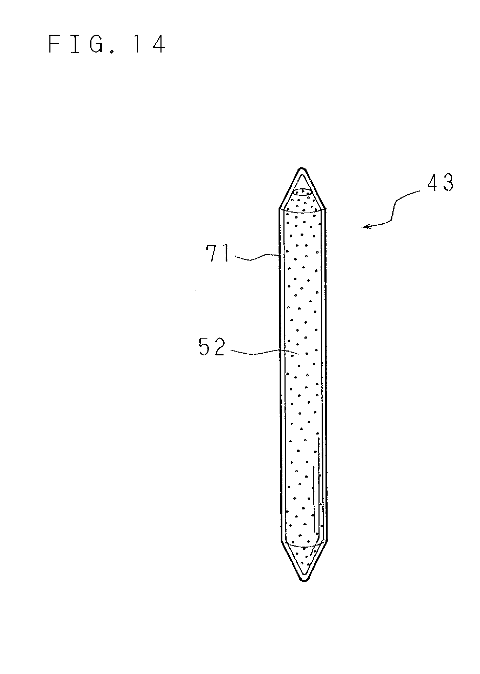

FIG. 5 is a partially-broken perspective view showing that the deodorizing bars shown in FIG. 14 are attached in the four corners of a room;

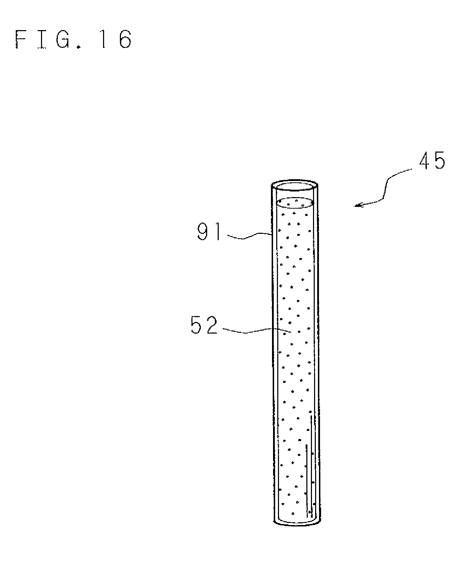

FIG. 16 is a perspective view showing a cleaning bar of the invention;

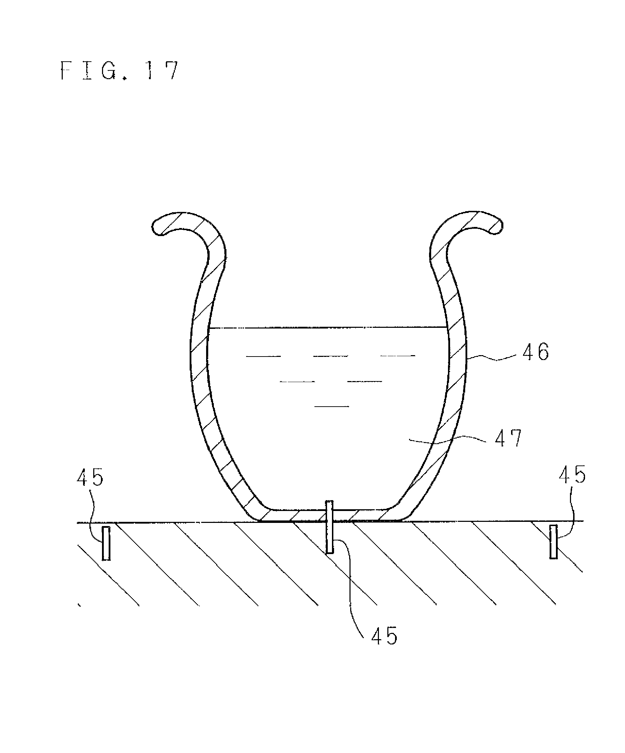

FIG. 17 is a longitudinally sectional view which shows that the cleaning bar shown in FIG. 16 is inserted into a pot of an unglazed pottery; and

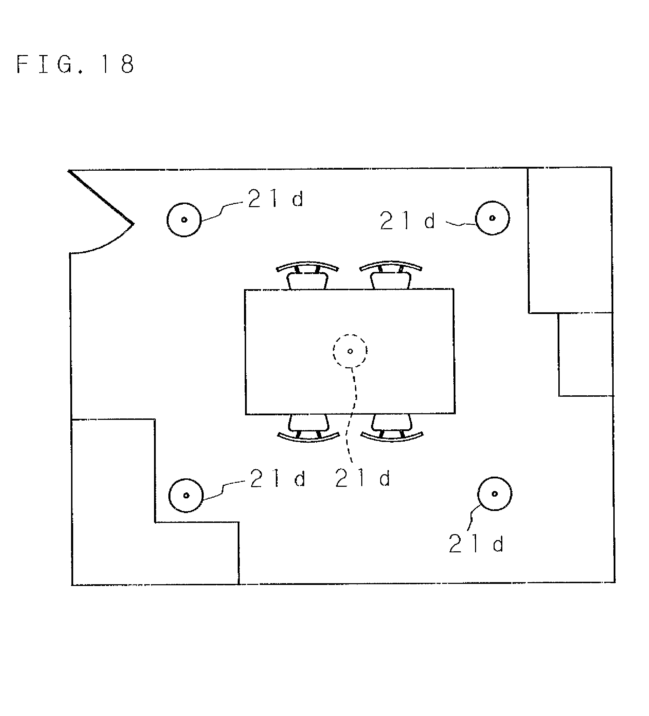

FIG. 18 is a plan view of glass containers of cone disposed indoors.

Hereinafter, the present invention will be described in detail, in reference to the drawings showing embodiments.

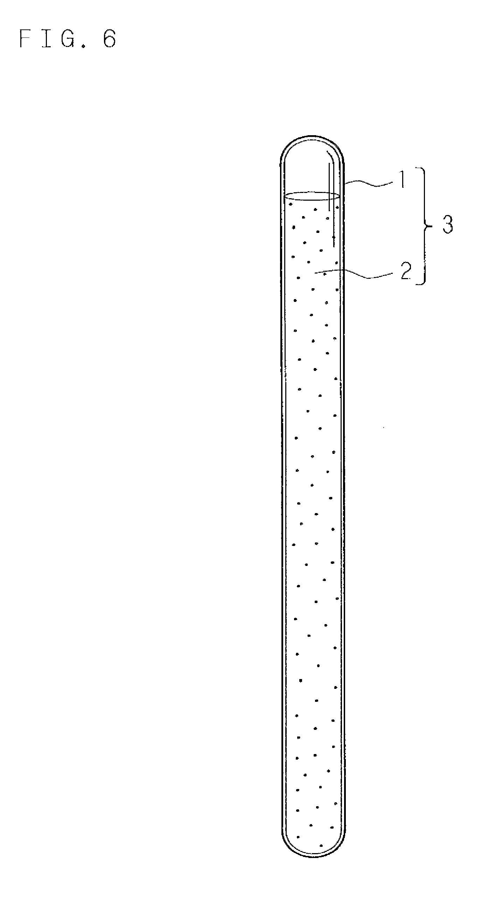

FIG. 6 is a schematic view showing an electrification preventing implement of the present invention. In a Pyrex tube (reinforced glass tube) 1, 500 to 600 g of granule or powder of, for example, silicon (Si) 2 is tightly sealed. The silicon 2 is previously placed in a minus-ionized quartz crucible for a predetermined time to be minus-ionized.

An appropriate hole is dug nearly in the center of a house, a factory, or a farm. One electrification preventing implement 3 is vertically embedded in the hole.

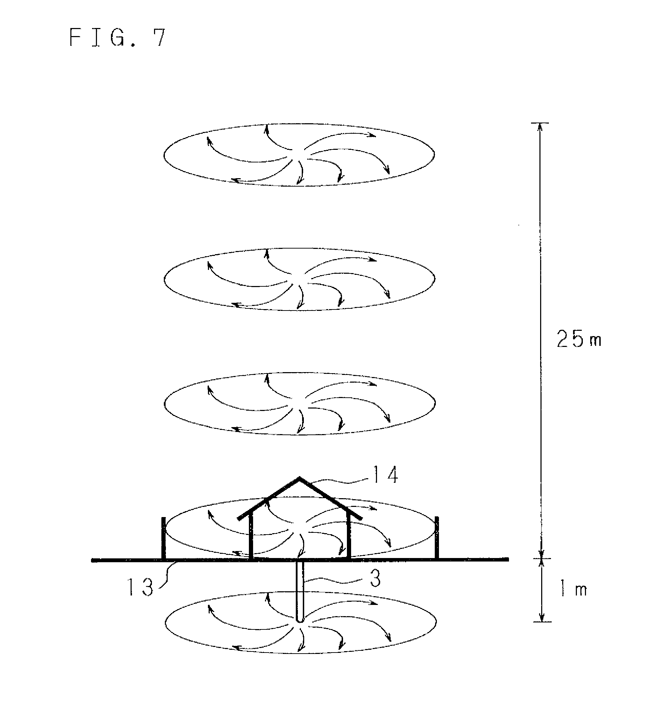

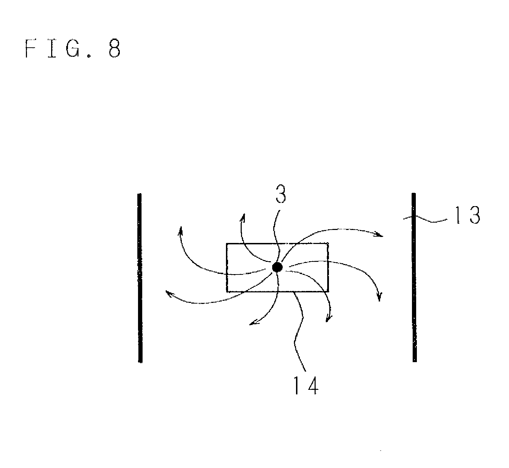

FIG. 7 is a side view of the house in which the electrification preventing implement 3 is embedded and its neighborhood. FIG. 8 is a plan view of the house and its neighborhood. When flow current is measured using a tester to quantify static electricity in the land 100 m above the sea level, the result was 30 to 40 mu A. The static electricity was reduced to about 15 mu A by the embedment of the electrification preventing implement 3. Probably, this is because the stray current on the ground surface and its neighborhood was allowed to flow easily and the residing static electricity was discharged to be eliminated. The embedment of the implement 3 reduces the intensity of current flowing human body, inhibiting the above-mentioned hindrances to men's healthy and morbid symptoms.

With the clockwise eddy of electric current as shown in FIGS. 7 and 8, minus ions from the silicon 2 so act that the concentration of minus ions in the house and its neighborhood is increased. It is generally known that the minus ions are effective in recovery from fatigue, cleaning of blood, and so forth. Air cleaning implements, bedding (futon, pillows), and so forth having such functions have been widely used. With the electrification preventing implement 3 embedded, minus ions from the silicon 2 activates hydrogen molecules. Thus, a comfortable living space in which the air is cleaned can be obtained. As to human bodies, effects whether they are physical or mental can be appreciated. For example, a person feels himself agile, becomes more patient, and so forth.

According to the method of this invention, a hole for embedding only one electrification preventing implement 3 is merely dug. The method can be carried out more easily as compared with conventional embedment of carbon. As only one glass tube is embedded, the implement can be easily transported.

In a fish feed factory filled with bad smells, the bad smells disappeared immediately after the electrification preventing implement 3 was embedded.

When the implement 3 was applied to a farm, plants were activated, and the growth rate was enhanced.

When the three to six implements 3 are embedded in a lake, the water is cleaned.

It is verified that the effects of the electrification preventing implement 3, which has a length of 1 m, extends to the space about 25 m above the ground level. In the case of a length of 1.5 m, the effects extend to the space about 50 m above the ground level. Thus, the length and the diameter of the Pyrex tube 1, and the amount of the silicon 2 can he appropriately selected, depending on lands and buildings. The effects are long-lasting. A plurality of implements 3 may be embedded. The larger number of the implements 3 is, the larger effect is obtained.

Though Si is used in the above embodiments, SiOx may be used. With component of SiOx, good effects can be obtained provided that x is a number of from 1.00 to 1.95.

Higher effects can be obtained as the ionization degree is higher.

As abovementioned, the embedment of a glass bar including silicon therein allows stray current to flow easily under and above ground. This can prevent electrification having a bad influence on a human body, animals, plants and objects.

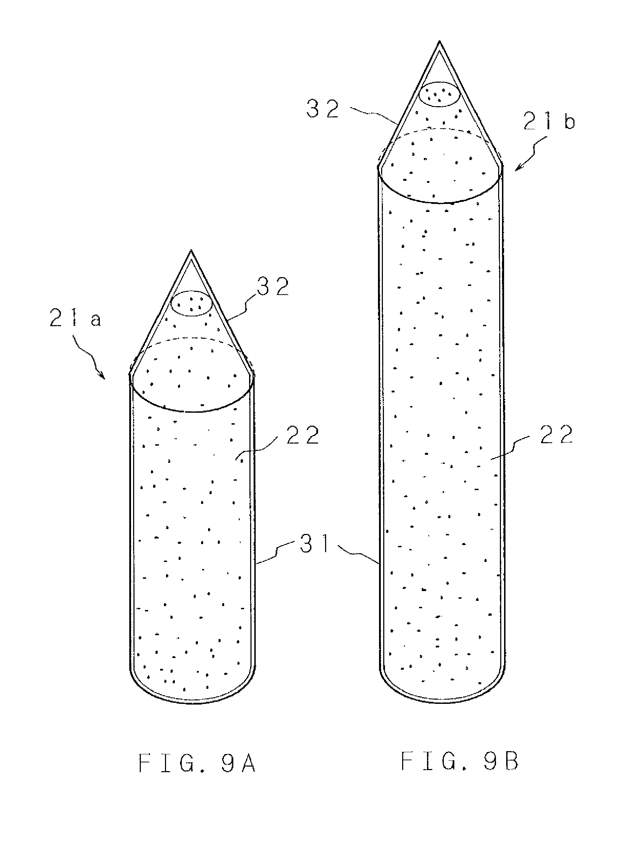

FIGS. 9A and 9B are perspective views showing space activating implements according to the invention. In the figure, numerals 21 and 21b designate a glass container comprising a columnar portion 31 and a conical portion 32 provided on the columnar portion 31. For the glass container 21a shown in FIG. 9A, the columnar portion 31 has a diameter of 2 cm and a height of 3 cm. The conical portion 32 provided on the columnar portion 31 has a height of 2 cm. The overall height is 5 cm. For the glass container 21b shown in FIG. 9B, the columnar portion 31 has a diameter of 2 cm and a height of 5 cm. The conical portion 32 provided on the columnar portion 31 has a height 2 cm. The overall height is 7 cm.

In the glass containers 21a and 21b, granule and powder of, for example, silicon (Si or SiOₓ) 22 is filled. The silicon 22 is previously placed in a minus-ionized quartz crucible for a predetermined time to be minus-ionized.

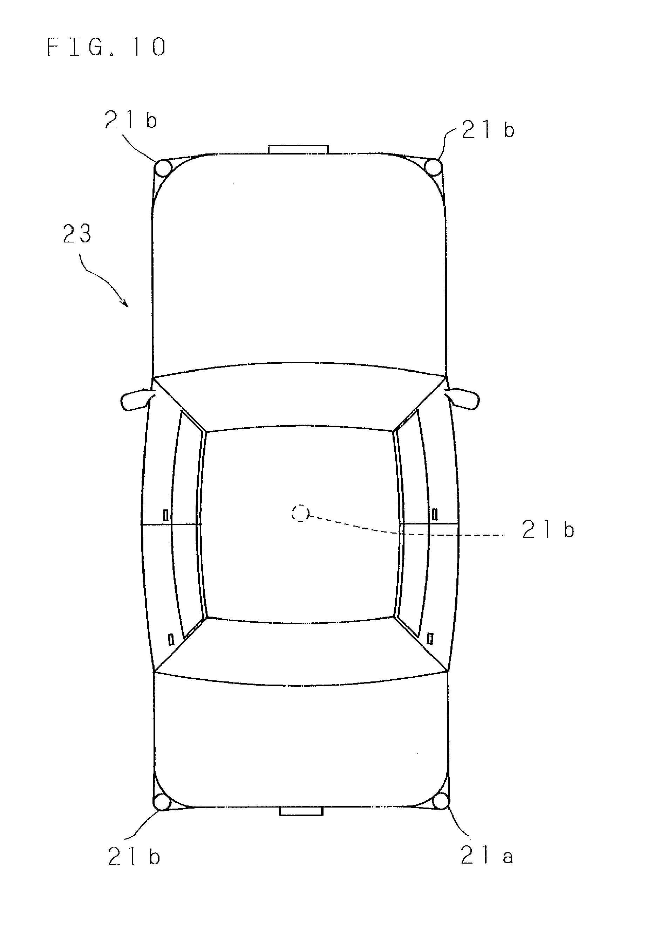

FIG. 10 is a plan view of the glass containers 21a and 21b mounted in a motorcar. The glass containers 21a in which the silicon 22 is filled are attached to the four corners in the periphery of a motorcar 23 to be fixed. The glass container 21b containing the silicon 22 filled therein is mounted nearly to the center inside of the motorcar to be fixed. Then, a tuning fork (for example, 885 Hz) is rung, and rotated anti-clockwise (opposite to the rotation of the earth) five times, and then rotated clockwise (in the same direction as the rotation of the earth) five times.

Thereby, it is verified that a harrier is formed to a height of 7 m in the space defined in a plane by the glass containers 21a arranged in the four corners. Minus ions emitted from the tops of these glass containers 21a and the glass container 21b mounted in the center inside of the motorcar activates the air, persons, and objects present in the space. With the activated air, persons in the motorcar have subjective symptoms such as invigorating feeling, health recovery, tranquilly, increased strength of body, improvements in sense of sight and hearing, and so forth. It is presumed that the minus ions act directly on the human bodies to be introduced.

As a prominent phenomenon caused by the activation of an object, the improvement in fuel consumption is exemplified. To a motorcar, a power is given ordinarily by combustion of hydrocarbon type fuels such as gasoline and so forth in the engine The combustion process includes the chemical chain-reactions which proceed rapidly. A high energy is required to break the covalent bonds of hydrocarbons, oxygen, and hydrogen as reactants in the combustion. When the covalent bonds are cut, these compounds are dissociated into some kinds of free radicals or atoms in the active state. In these steps, various intermediates (hydrogen peroxide and so forth) are produced and carry out their contact action with each other. In the case of perfect combustion, carbon dioxide and water are produced.

It is presumed that when the gasoline is activated with the minus ions emitted from the silicon 22, the hydrocarbon is ready to be in the active sate, which enhances the consumption efficiency

In general, the thermal efficiency of a reciprocating gasoline engine, which is typical of internal combustion engines, is 25 to 30%. For diesel engines, the thermal efficiency is generally estimated to be about 30%. In a mechanism for converting heat energy to mechanical energy, the overall heat loss including radiation loss, friction loss, and other losses amounts to 70 to 75 %. Therefore, there is a great possibility that the fuel consumption is considerably decreased by reducing this loss by some means. Since the energy to be consumed in the above combustion process is given with the minus ion emitted from the silicon 22 in the method of the invention the heating loss is reduced, thereby improving in fuel consumption. Practically, the inventors identified the improvement in fuel consumption by at least 20%. The results are shown in Tables 1 and 2. The results in Table were obtained, using a automatic transmission motorcar. Those in Table 2 were obtained with a five-step manual transmission motorcar. The improvement in fuel consumption realizes the inhibition of environmental pollution and the effective use of natural resources.

### tables 1 and 2

Also, in the car with the implements, running stability is superior, in particular high speed (120 - 150 km/h) straight running stability is good. In addition cornering stability in the curve such as in interchange of a high way is improved.

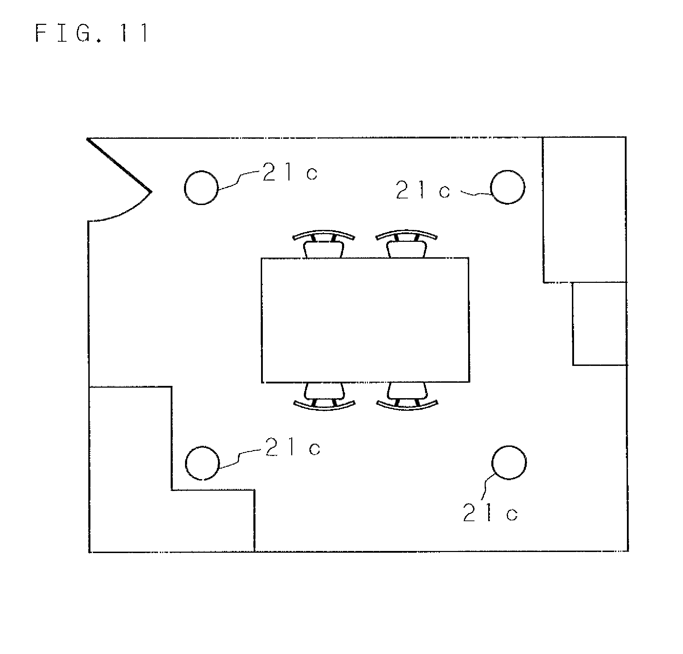

FIG. 11 is a plan view of glass containers 21c disposed indoors. In a room of a house, the glass container 21c which contains a columnar portion 31 with a diameter of 5 cm, a height of 7 cm and a conical portion 32 with a height of 3 cm, and in which the silicon 22 is filled are arranged in the four corners of an optional rectangular area. Then, a tuning fork (for example, 885 Hz) is rung, and rotated anti-clockwise (opposite to the rotation of the earth) five times, and then rotated clockwise (in the same direction as the rotation of the earth) five times.

In this space as well as in the motorear, such effects that the air, living things, and objects are activated can be obtained. For example, the rotting rate of a food (meat) placed in the space is reduced. That is, freshness maintaining effects can be obtained. In the case that the food is once placed in the space and later placed outdoors, the same effects as those given when the food is held indoors all the time can be obtained. A metal placed in the space is inhibited from being corroded and oxidized, and is given a gloss.

In the embodiments shown in FIGS. 9A and 9B, the glass container has a columnar portion and a conical portion. However, this invention is not restricted onto the form. A combination of a prism and a pyramid is included. Moreover, a cone, a three-sided pyramid, and a four-sided pyramid as a whole shape are included. In other words, any shape is included provided that it contains a point portion at the top thereof.

When four glass containers 21d of cone (diameter 10 cm, height 10 cm) containing silicon are disposed near the corners of a room and one glass containers 21d of cone is near the center as shown in FIG. 18, a similar effect is obtained without sounding the tuning fork.

In a case where four glass containers of cone (diameter 1 cm, height 1 cm) containing silicon were disposed around a car and one glass container of cone was near the center under the car, the car could be lifted up by six average men.

The silicon 22 may be metal silicon. In the case of SiOx used as the silicon 22, good effects can be obtained provided that x is a number of from 1.00 to 1.95. Higher effects can be obtained as the ionization degree is higher. The times of rotation of the tuning fork is not restricted to five.

As above, glass containers including silicon therein are disposed, and sounding body is rung in the invention. Then, minus ions emitted from the silicon are filled into the space defined by the at least three space activating implements to form a barrier. The minus ions act on the air, living things such as human bodies, plants, animals, and so forth, and objects to activate. With waves generated from the sounding body, the activation state is maintained. Therefore, the space can be activated without using electricity.

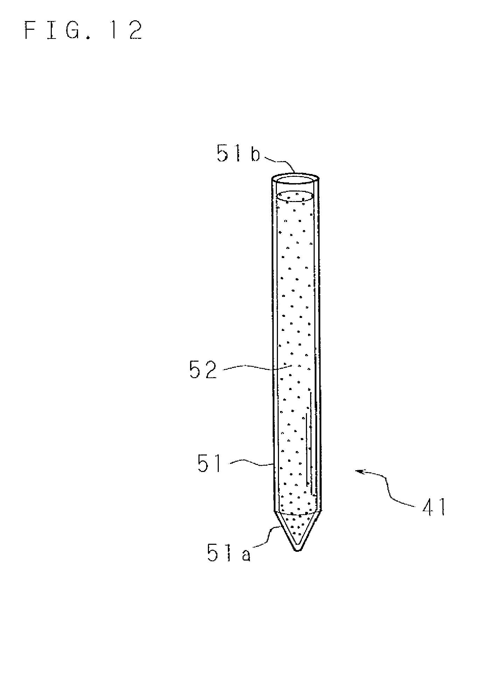

FIG. 12 is a schematic view showing a cleaning/deodorizing bar according to the invention. The cleaning/deodorizing bar 41 has a glass tube 51 with a diameter of.3 mm and a length of 3 cm in which granule and/or powder (grain size DIAMETER 0.03 to 2.3 mm) of, for example, silicon 52 (Si or SiOx (0<x</=2) is sealed. One end of the glass tube 51 is so worked as to have a substantially conical shape (conical portion 51a), and the other end is so worked as to be plane (plane portion 51b). Silicon 52 is previously placed in a quartz crucible minus ionized, for a predetermined time (about 5 minutes) to be minus ionized.

From the silicon 52, minus ions are emitted. It is presumed that the minus ions activate the environmental, hydrogen. The minus ions emitted through the plane top end of the tube 51 acts on water. The minus ions emitted through the conical portion of the glass tube 51 acts on smells.

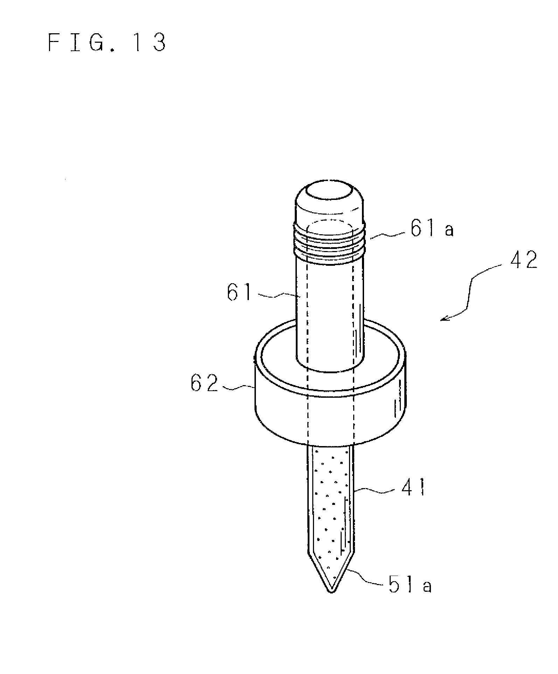

FIG. 13 is a perspective view which shows a state that the cleaning/deodorizing bar 41 shown in FIG. 12 is attached to a valve at a tap of a water supply. In the valve 42, to the head portion of a screw 61 having a screw portion 61a in the lower portion thereof, a rubber 62 is fitted. The valve 42 has a through-hole which opens axially in the leg portion of the screw 61. Into the through-hole, the cleaning/deodorizing bar 41 is inserted. As shown in FIG. 13, the cleaning/deodorizing bar 41 is so inserted into the through-hole that the plane portion 51b of the cleaning/deodorizing bar 41 protrudes on the head portion side of the screw 61.

As described above, minus ions emitted from the cleaning/deodorizing bar 41 can act on water and smells. Therefore, water having no bad taste and smells of chlorine, chlorinated lime, and so forth can be obtained through the tap of a water supply to which the cleaning/deodorizing bar 41 is attached.

Water supplied through a valve having the cleaning/deodorizing bar 41 of the invention and through a valve to which the cleaning/deodorizing bar 41 was not attached in a house where the implement 3 shown in FIG. 7 is embedded was inspected according to Waterworks Law. The results will be described. In the case of no cleaning/ deodorizing bar 41 attached, the water had a number of general bacterial of not less than 300/ml and was positive for Escherichia coli. In the case of the bar 41 attached, the water had a number of general bacteria of 0 and was negative for Escherichia coli.

The river water had COD (chemical oxygen demand) of 10 to 20. In the case of the cleaning/deodorizing bar 41 used, the COD was significantly reduced to 0 to 5.

FIG. 14 is a schematic view showing a deodorizing bar of the present invention. The deodorizing bar 43 has a glass tube 71 with a diameter of 3 mm and a length of 3 cm in which granule and/or powder (grain size DIAMETER 0.03 to 2.3 mm) of, for example, silicon 52 ( Si or SiOx (0<x</-2)) is sealed. The opposite ends of the glass tube 71 is so worked as to have a substantially conical shape. The silicon 52 is previously placed in a minus-ionized quartz crucible for a predetermined time (about 5 minutes) to be minus-ionized.

From the silicon 52, minus ions arc emitted. It is presumed that the minus ions activate the environmental hydrogen. As the opposite ends of the glass tube 71 have a conical shape, the minus ions act on smells and wind.

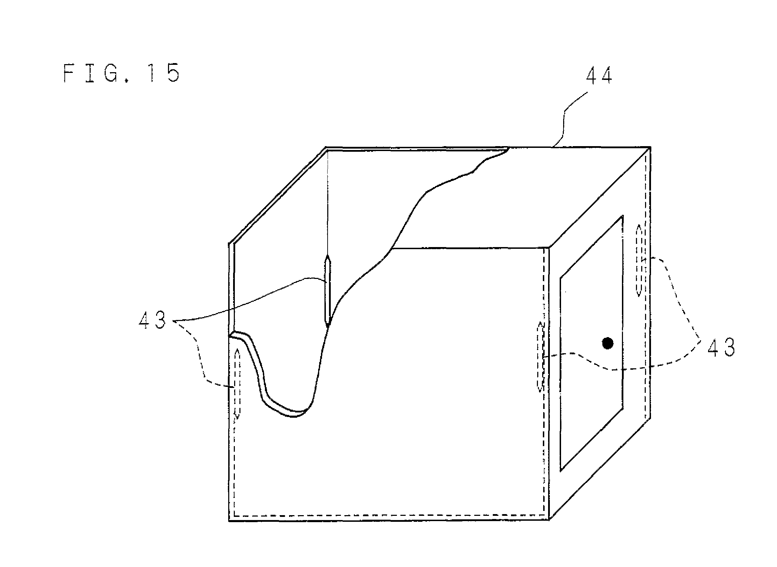

FIG. 15 is a partially-broken perspective view showing that the deodorizing bars 43 shown in FIG 14 are attached in the four corners of a room 44. Minus ions emitted from the silicon 52 of deodorizing bars 43 eliminates the plus electrostatic charge from the air of the room 44 to remove the smells in the room,

In the case that a campfire is held, the deodorizing bars 43 arc embedded to a depth of about 3 cm in such positions as to surround the fire, Participants on the outside of the place surrounded by the deodorizing bars feel no irritation in their eyes if smoke touches their eyes. In addition, the difference between the temperatures on the outside, inside of the place surrounded by the deodorizing bars is increases.

FIG. 16 is a schematic view showing a cleaning bar embodying the invention. The cleaning bar 45 has a glass tube 91 with a diameter of 3 mm and a length of 3 cm in which granule and/or powder (grain size DIAMETER 0.03 to 2.3 mm) of, for example, silicon 52 (Si or SiOx (0<x </.-2)) is sealed. The opposite ends of the glass tube 91 is so worked as to be plane. The silicon 52 is previously placed in a minus-ionized quartz crucible for a predetermined time (about 5 minutes) to be minus ionized.

From the silicon 52, minus ions are emitted. It is presumed that the minus ions activate the environmental hydrogen The minus ions emitted through the plane top ends of the glass tube 91 act on water and earth

FIG. 17 a longitudinally sectional view which shows that the cleaning bar 45 shown in FIG. 16 is inserted into a pot of an unglazed pottery for use. The pottery 46 is placed on the earth and contains water 47 therein. In addition, a plurality of cleaning bar 45 are embedded in 6 - 9 cm depth vertically, for example, four bars 45 are disposed around the pottery 46. Though two bars 45 may be arranged around the pottery 46, the larger number is, the larger effect is obtained.

As described above, as minus ions emitted from the bar 45 act on the water and the earth, taste and smells of the residual chlorine can be removed from the water in the pottery 46.

The cleaning bar 45 shown in FIG. 16 can be applied to trees. For example, the cleaning bar 45 is embedded near the roots of a tree to increase the number of microorganisms and promote the vitality of the tree.

In the case of SiOx used as the silicon 52, good effects can be obtained provided that x is a number of from 1.00 to 1.95. Especially, it has been founded that the best effects can be obtained when x is a number of 1.5. It is desirable that the silicon 52 is minus ionized. Higher effects can be obtained as the ionization degree is higher.

As above, in a cleaning/deodorizing bar of the invention a predetermined element or compound is sealed in a glass tube. Minus ions emitted from the element or compound removes static electricity to clean water, soil and air.

In all examples, silicon is sealed in the glass container. However, it has been found that element such as C, Al, P, Ge, Sn, Pb, Cu, Ag, Au, Ni, or Fe may be used to give effects. In this case, it is desirable that the respective elements are minus ionized in the quartz crucible before they are sealed in the glass container.

As this invention may be embodied in several forms without departing from the spirit of essential characteristics thereof, the present embodiments are therefore illustrative and not restrictive, since the scope of the invention is defined by the appended claims rather than by the description preceding them, and all changes that fall within metes and bounds of the claims, or equivalence of such metes and bounds thereof are therefore intended to be embraced by the claims.

**TABLE 1**

| | SPEED (km/h) | RUNNUNG DISTANCE (km) | FUEL VOLUME CONSUMED (c c) | FUEL CONSUMPTION (km/l) | EFFECT |
|---|---|---|---|---|---|
| EQUIPPED | 60 | 49.3 | 2870 | 17.18 | 34.5%up |
| UN-EQUIPPED | | 49.2 | 2125 | 23.11 | |
| EQUIPPED | 100 | 49.1 | 3180 | 15.44 | NO CHANGE BECAUSE OF RAIN |
| UN-EQUIPPED | | 49.1 | 3150 | 15.59 | |

**TABLE 2**

| | SPEED (km/h) | RUNNUNG DISTANCE (km) | FUEL VOLUME CONSUMED (cc) | FUEL CONSUMPTION (km/l) | EFFECT |
|---|---|---|---|---|---|
| EQUIPPED | 60 | 48.8 | 2300 | 21.22 | 33.4%up, |
| UN-EQUIPPED | | 48.8 | 1725 | 28.30 | |
| EQUIPPED | 70 | 48.7 | 2700 | 18.00 | NO CHANGE BECAUSE OF STORM |
| UN-EQUIPPED | | 48.8 | 2600 | 18.76 | |
| EQUIPPED | 100 | 48.7 | 3150 | 15.46 | 26.0 % up |
| UN-EQUIPPED | | 48.7 | 2500 | 19.48 | |
| EQUIPPED | 120 | 48.8 | 3840 | 12.70 | NO CHANGE BECAUSE OF STORM |
| UN-EQUIPPED | | 48.8 | 3780 | 12.69 | |

## Claims

1. A cleaning/deodorizing bar (41,43),
wherein a predetermined granular and/or powdery element and/or compound is scaled in a glass tube (51,71) whose at least one end is substantially conical, said element and/or compound being minus-ionized.

2. The cleaning/deodorizing bar (41) according to claim 1,
wherein the other end of the glass tube (51) is substantially plane.

3. The cleaning/deodorizing bar (41) according to claim 1 or 2, wherein said element and/or compound is Si and/or SiOₓ (52) (0<x≤2).

4. The cleaning/deodorizing bar (41) according to claim 1 or 2,
wherein said element is selected from the group consisting of C, Al, P, Ge, Sn, Pb, Cu, Ag, Au, Ni and Fe.

5. The deodorizing bar (43) according to claim 1,
wherein the other end of the glass tube (71) is substantially conical.

6. The deodorizing bar 43 according to claim 5, wherein said element and/or compound is Si and/or SiOₓ (52) (0<x≤2).

7. The deodorizing bar (43) according to claim 5, wherein said element is selected from the group consisting of C, Al, P, Ge, Sn, Pb, Cu, Ag, Au, Ni and Fe.

8. A cleaning bar (45) wherein a predetermined granular and/or powdery element and/or compound is sealed in a glass tube (91) whose ends are substantially plane, said element and/or compound being minus-ionized.

9. The cleaning bar (45) according to claim 8,
wherein said element and/or compund is Si and/or SiOₓ (52) (0<x≤2).

10. The cleaning bar (45) according to claim 8,
wherein said element is selected from the group consisting of C, Al, P, Ge, Sn, Pb, Cu, Ag, Au, Ni and Fe.
